# EUROPEAN PATENT APPLICATION

(11) **EP 3 092 960 A1**
(43) Date of publication of application: **16.11.2016**
(21) Application number: 16169578.8
(22) Date of filing: 13.05.2016
(51) Int. Cl.: A61B 17/3203, A61B 90/57, A61M 5/14

(54) **SUPPORT TOOL, OPERATION SECTION, AND LIQUID EJECTION DEVICE**

(30) Priority: 15.05.2015 JP 2015099785
(71) Applicant: Seiko Epson Corporation, Tokyo 160-8801 (JP)
(72) Inventor: Ide, Katsuya, Suwa-shi, Nagano 392-8502 (JP)
(74) Representative: Hoffmann Eitle

(57) **Abstract**

A support tool (80) that is used with a liquid ejection device (10) ejecting liquid and including an operation section (50) operated by an operator and a device main body section (20) and is capable of supporting a plurality of cylindrical members (12,14, 18) including a tube group connected to the operation section (50) and the device main body section (20), the support tool (80) including a first support section (82a) capable of supporting a first cylindrical member (14) among the plurality of cylindrical members in such a way that the first cylindrical member (14) is immobile in the axial direction thereof and second support sections (82b, 82c) capable of supporting second cylindrical members (12, 18) different from the first cylindrical member (14) in such a way that the second cylindrical members are movable in the axial directions thereof, wherein the first support section (82a) and the second support sections (82b, 82c) are integrated with one another.

## Description

### BACKGROUND

### 1. Technical Field

The present invention relates to a support tool that supports cylindrical members including a tube group connected to an operation section that ejects liquid, an operation section including the support tool, and a liquid ejection device including the operation section.

### 2. Related Art

In recent years, in a medical field, such as an incision and removal of living tissue, a liquid ejection device is used because many advantages, such as an ability to maintain the surface of living tissue at a surgical operation site at a low temperature and no injury of blood vessels and bodily parts, attract attention. A liquid ejection device ejects liquid, such as physiological saline, in the form of pulsed flow toward a living body to excise or fragmentate living tissue. The liquid ejection device includes a handpiece (hereinafter referred to as an operation section) including a nozzle through which the liquid is ejected and a device main body section including a liquid supply section, a suction device, a controller, and other components. A plurality of tubes (hereinafter referred to as a tube group) including a supply tube through which the liquid is supplied, a suction tube through which ejected liquid and fragmentated tissue are sucked, and a cable for generating the pulsed flow are connected between the operation section and the device main body section (see JP-A-2014-206088, for example).

As medical treatment advances, a variety of kinds of biological sites become targets of the liquid ejection device, and more accurate medical practice is required. Some types of medical practice require a microscope or a magnifying glass. A practitioner performs medical practice with the operation section described above held by the practitioner's hand. The supply tube, the suction tube, and the cable, which form the tube group connected to the operation section, differ from one another in terms of rigidity depending on the characteristics of the tubes and the state of the liquid passing through the tubes. Further, a lengthy tube is as long as several meters. Therefore, when the practitioner operates the operation section, the tubes that form the tube group connected to the operation section bend at different values of curvature and behave in different manners, resulting in reaction force produced at the operation section and undesirably making accurate operation difficult.

### SUMMARY

An advantage of some aspects of the invention is to solve at least a part of the problems described above, and the invention can be implemented as the following forms or application examples.

### Application Example 1

This application example is directed to a support tool that is used with a liquid ejection device including an operation section operated by an operator and a device main body section and supports a plurality of cylindrical members including a tube group connected to the operation section and the device main body section. The support tool includes a first support section capable of supporting a first cylindrical member among the plurality of cylindrical members in such a way that the first cylindrical member is immobile in an axial direction thereof and second support sections capable of supporting second cylindrical members different from the first cylindrical member in such a way that the second cylindrical members are movable in axial directions thereof, wherein the first support section and the second support sections are integrated with one another.

According to the configuration described above, in a state in which one of tubes in the tube group, that is, one of cylindrical members including tubes and cables is set to be a first cylindrical member, the first cylindrical member can be supported by a corresponding support section in a state in which the first cylindrical member is immobile in the axial direction thereof, and the other cylindrical members can be supported by corresponding support sections in a state in which the cylindrical members are movable in the axial directions thereof. Therefore, even the plurality of cylindrical members different from one another in terms of rigidity and behavior basically curve and behave in accordance with the curvature and behavior of the first cylindrical member. As a result, differences in the curvature and behavior of the cylindrical members that occur when a practitioner operates the operation section can be reduced, whereby variation in reaction force produced by the plurality of cylindrical members can be reduced. A liquid ejection device including the operation section having the support tool that allows accurate operation with excellent operability can therefore be provided.

### Application Example 2

In the support tool described above, the first cylindrical member may be at least one of a tube or a cable having a greatest outer diameter and a tube or a cable having highest rigidity in the tube group.

According to the configuration described above, selecting a large-diameter tube or cable and/or a high-rigidity tube or cable as the first cylindrical member allows the second cylindrical members supported by the support tool to readily follow the curvature and behavior of the first cylindrical member. As a result, differences in the curvature and behavior of the cylindrical members that occur when the practitioner operates the operation section can be reduced, whereby variation in reaction force produced by the plurality of cylindrical members can be reduced.

### Application Example 3

In the support tool described above, the first support section may have a first opening through which the first cylindrical member is inserted, each of the second support sections may have a second opening through which the corresponding second cylindrical member is inserted, an inner diameter of the first support section may be smaller than an outer diameter of the first cylindrical member, and an inner diameter of each of the second support sections may be greater than an outer diameter of the corresponding second cylindrical member.

According to the configuration described above, the first cylindrical member is inserted through the first opening and supported by the first support section with the first cylindrical member being immobile in the axial direction thereof, and the other second cylindrical members are inserted through the corresponding second openings and supported by the second support sections with the second cylindrical members being movable in the axial directions thereof.

### Application Example 4

In the support tool described above, a second opening may be formed of a first portion and a second portion of the corresponding second support section, a protrusion extending in a direction away from the second portion may be formed in the first portion, and a surface of the protrusion may form an inclined surface extending toward the second opening in a direction in which the corresponding second cylindrical member is inserted.

According to the configuration described above, at least one of the second support sections has a protrusion (hereinafter referred to as a guiding member) having an inclined surface that guides the corresponding second cylindrical member to the second opening when the second cylindrical member is inserted. Therefore, when the operator uses the operation section of the liquid ejection device, a specific tube or cable can be readily attached or detached to or from the corresponding second support section. As a result, when the operation section is exchanged or otherwise handled, the operation section can be readily attached or detached to or from the liquid ejection device. A liquid ejection device including the operation section having the support tool with excellent operability can therefore be provided.

### Application Example 5

In the support tool described above, the first cylindrical member may be part of the liquid ejection section, and the second cylindrical members may be formed of the tube group.

According to the configuration described above, the support tool is fixed to the position of a component of the operation section or the device main body section, that is, a predetermined position. The support tool can support tubes and/or cables in that position in such a way that they are movable in the axial directions thereof but immobile in the radiation directions thereof. Therefore, variation in the positions of the tubes in the tube group in the radial direction can be suppressed and a situation in which the tubes come apart in the radial direction can be avoided. As a result, differences in the behavior of the tubes in the tube group can be reduced, whereby variation in reaction force produced by the plurality of tubes in the tube group can be reduced. A liquid ejection device including the operation section having the support tool that allows accurate operation with excellent operability can therefore be provided.

### Application Example 6

In the support tool described above, the first cylindrical member may have a cylindrical portion having an outer circumferential portion around which a grooved section is formed, and the first support section may have a locking section fittable to the grooved section.

According to the configuration described above, the arcuate support section of the support tool is fit in the grooved section provided around the circumference of a component that is a cylindrical member to achieve a state in which the component is movable along the grooved section in the circumferential direction but immobile in the axial direction. Further, when the locking section, which protrudes inward from the support section, engages with locking reception sections, which are formed at predetermined intervals in the grooved section of the component, the support tool can be held in a predetermined position in the circumferential direction. The support tool can therefore support the tubes or cables with a radial load reduced even in a state in which the tubes or cables are arranged in an arbitrary position relative to the component.

### Application Example 7

This application example is directed to an operation section that is used with a liquid ejection device that ejects liquid and operated by an operator. The operation section includes a gripper section, a tubular nozzle section that is provided in the gripper section and ejects the liquid, a tube group which is connected to the gripper section and through which the liquid flows, and one of the support tools described above, which supports at least one tube in the tube group.

According to the configuration described above, an operation section that has excellent operability and allows an ejection drive section to cause liquid supplied from a liquid supply mechanism to be ejected through the nozzle section for medical practice and liquid containing fragmentated living tissue to be sucked at desired suction force in accordance with a situation of a medical treatment target site and therearound can be provided.

### Application Example 8

This application example is directed to a liquid ejection device including the operation section described above, an ejection drive section that ejects liquid through a nozzle section provided in the operation section, a liquid supply mechanism that supplies the ejection drive section with the liquid, a liquid suction mechanism capable of sucking the liquid ejected through the nozzle section, a device main body section that outputs a drive signal to the ejection drive section, and a cable connected between the ejection drive section and the device main body section.

According to the configuration described above, a liquid ejection device including the operation section that has excellent operability and allows the ejection drive section to cause liquid supplied from the liquid supply mechanism to be ejected through the nozzle section for medical practice and liquid containing fragmentated living tissue to be sucked at desired suction force in accordance with a situation of a medical treatment target site and therearound can be provided.

### BRIEF DESCRIPTION OF THE DRAWINGS

The invention will be described with reference to the accompanying drawings, wherein like numbers reference like elements.
Fig. 1 is a schematic view showing an overall configuration of a liquid ejection device.
Figs. 2A to 2C are schematic configuration diagrams showing an operation section.
Fig. 3 is a diagrammatic cross-sectional view showing the internal configuration of a pulsed flow applying part.
Figs. 4A and 4B describe a support tool according to a first embodiment.
Figs. 5A and 5B describe a support tool according to a second embodiment.

### DESCRIPTION OF EXEMPLARY EMBODIMENTS

Embodiments of the invention will be described below with reference to the drawings. In the drawings that the following description refers to, a member or a portion is drawn at an aspect ratio different from an actual aspect ratio in some cases for ease of description and illustration.

### Overall configuration of liquid ejection device

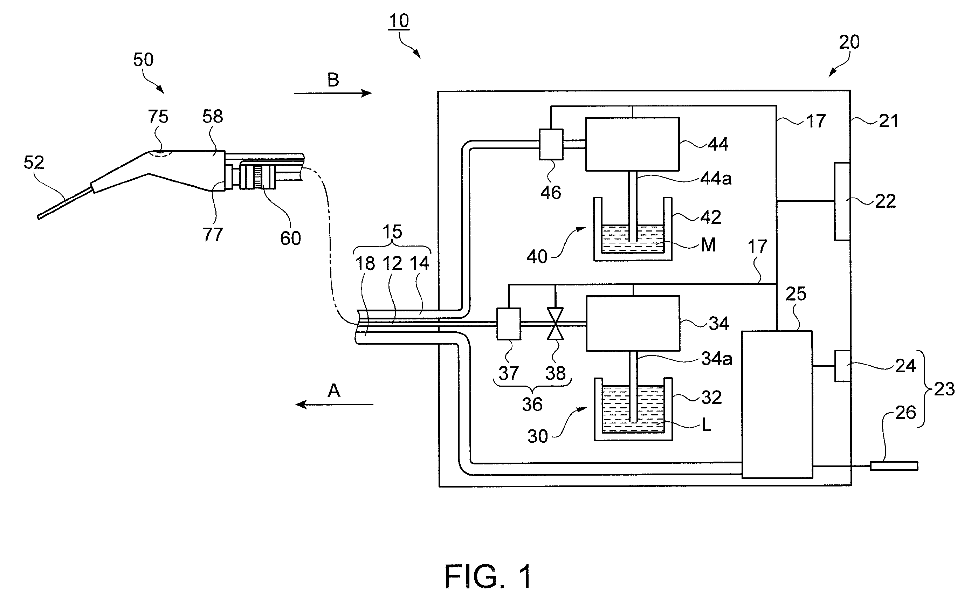

An overall configuration of a liquid ejection device 10 will be described with reference to Fig. 1. Fig. 1 is a schematic view showing the overall configuration of the liquid ejection device 10. The liquid ejection device 10 is primarily used for medical treatment and ejects a liquid L, for example, physiological saline and Ringer's solution, in the form of pulsed flow toward a medical practice target site to excise or fragmentate tissue. The liquid ejection device 10 includes a device main body section 20 and an operation section 50, as shown in Fig. 1. The device main body section 20 and the operation section 50 are connected to each other via a tube group 15.

### Device main body section

The device main body section 20 will first be described with reference to Fig. 1. The device main body section 20 includes a roughly box-shaped enclosure 21 and accommodates a liquid supply mechanism 30, a liquid suction mechanism 40, and a controller 25, as shown in Fig. 1.

The enclosure 21 is provided with a display section 22 and a switch section 23. The display section 22 is formed, for example, of a liquid crystal display and displays the quantity, the flow speed, and the pressure of the supplied liquid L ejected by the liquid ejection device 10 and other types of necessary information on the liquid L. The switch section 23 includes at least a power switch 24 and an ejection switch 26. The power switch 24 is a switch that activates the liquid ejection device 10. When the power switch 24 is flipped on, electric power is supplied to the device main body section 20. The ejection switch 26 is a switch that switches the action of the operation section 50 between ejection and no ejection of the liquid L therefrom. The ejection switch 26 is preferably formed, for example, of a foot switch operated with a practitioner's foot.

The liquid supply mechanism 30 has a function of supplying the operation section 50 with the liquid L to be ejected from the liquid ejection device 10 and includes a liquid storage 32, a supply pump 34, and a supply quantity adjuster 36, which are sequentially arranged along the flow of the liquid L (arrow A). The liquid storage 32 is what is called a liquid reservoir and stores the liquid L, such as physiological saline and Ringer' s solution, which is ejected from the liquid ejection device 10. Physiological saline and Ringer's solution, which hardly harm a living body, can be used in surgery.

The supply pump 34 can be, for example, a syringe-type pump or a tube pump. When a syringe-type pump is used, it is preferable to separately provide a device that supplies the liquid L into the syringe in consideration of continuous driving operation. A liquid acquisition tube 34a is attached to the supply pump 34, and an end portion of the liquid acquisition tube 34a is connected to the liquid storage 32. The supply pump 34 performs sucking action in the direction indicated by the arrow A to deliver the liquid L stored in the liquid storage 32 in the direction indicated by the arrow A.

The supply quantity adjuster 36 is provided in a halfway position along a supply tube 12, which is connected to the supply pump 34, and includes a supply flowmeter 37 and an electromagnetic valve 38. The supply flowmeter 37 measures the flow rate of the liquid L flowing through the supply tube 12. The supply flowmeter 37 can be, for example, a hot wire flowmeter or a turbine flowmeter. The electromagnetic valve 38 is a valve so controlled with an electric signal as to open and close, and opening and closing the valve controls the flow of the liquid L flowing through the valve. Specifically, the electromagnetic valve 38 instantaneously stops the flow of the liquid L at the time of malfunction, whereas stopping or starting the flow of the liquid L is performed at the time of medical practice or in other occasions. The supply pump 34 operates the electromagnetic valve 38 on the basis of a result of the measurement performed by the supply flowmeter 37 to adjust the flow rate of the liquid L flowing through the supply tube 12. The supply tube 12 is held by the operation section 50, which will be described later. The supply tube 12 will be described later in detail.

The liquid suction mechanism 40 has a function of sucking a liquid M containing part of fragmentated tissue left after the liquid L is ejected from the liquid ejection device 10 for medical practice. The liquid suction mechanism 40 further has a function of sucking excess liquid L used in the medical practice in order to ensure the field of vision of the practitioner during the medical practice. It is, however, assumed that the entire liquid sucked by the liquid suction mechanism 40 is referred to as the liquid M for ease of description.

The liquid suction mechanism 40 includes a suction flowmeter 46, a suction pump 44, and a drainage tank 42, which are sequentially arranged along the flow of the liquid M (arrow B). The suction flowmeter 46 has the same structure as that of the supply flowmeter 37 described above and is provided in a halfway position along a suction tube 14 held by the operation section 50, which will be described later. The suction flowmeter 46 measures the flow rate of the liquid M flowing through the suction tube 14. The suction pump 44 is not limited to a specific pump and can be, for example, a tube pump. The suction pump 44 performs sucking action in the direction indicated by the arrow B.

A drainage tube 44a is attached to the suction pump 44, and an end portion of the drainage tube 44a is connected to the drainage tank 42. The drainage tank 42 is what is called a liquid reservoir and stores the liquid M containing part of post-medical-practice fragmentated tissue sucked by the operation section 50 of the liquid ejection device 10. The liquid suction mechanism 40 can be further provided, as required, with a filter that is not shown but is provided along the flow path for removal of part of the fragmentated tissue.

The controller 25 is connected to the display section 22, the switch section 23, the liquid supply mechanism 30, and the liquid suction mechanism 40 described above via an internal cable 17 and oversees and controls the mechanisms described above. The controller 25 is connected to the operation section 50, which will be described later, via a cable 18, which forms the tube group 15.

### Operation section

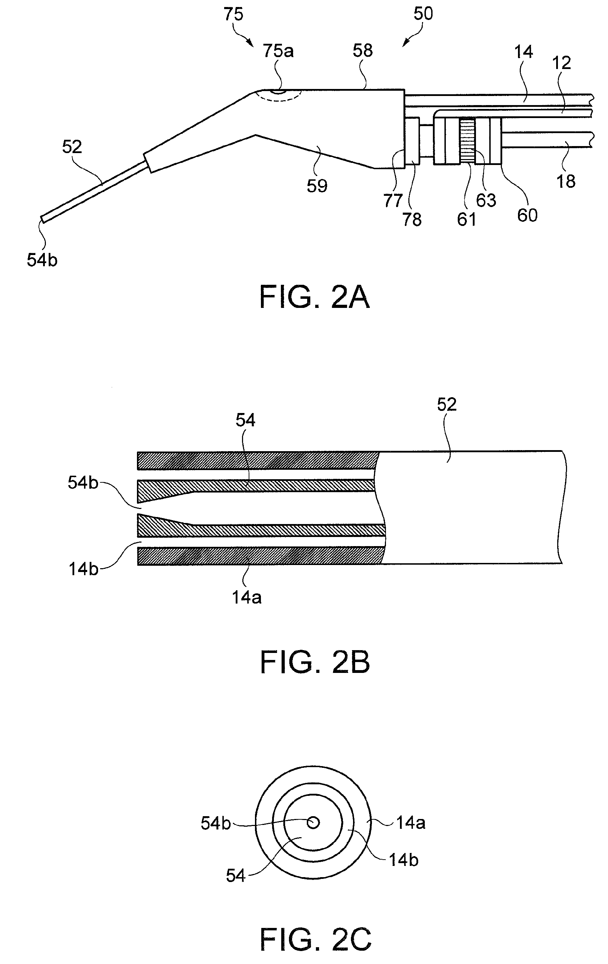

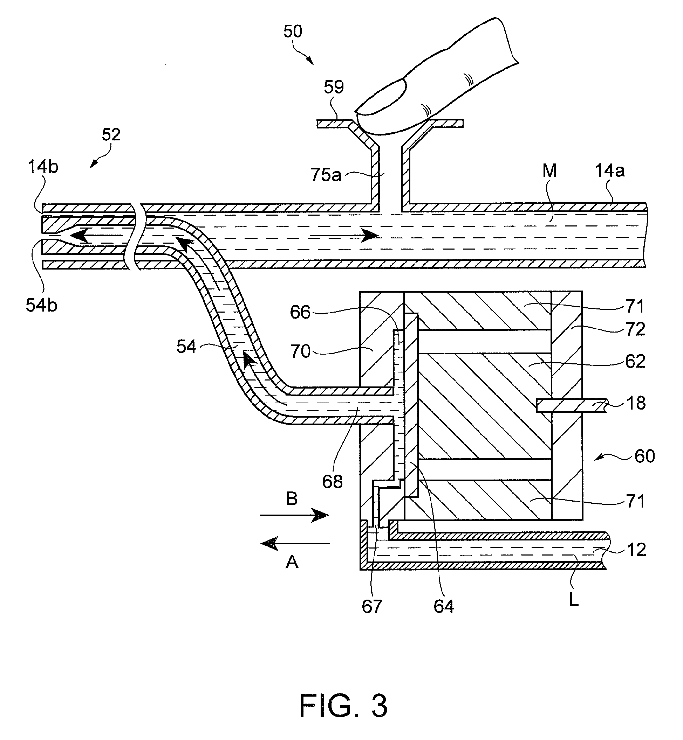

A summary of the operation section 50 will next be described with reference to Figs. 2A to 2C and Fig. 3. Figs. 2A to 2C are schematic configuration diagrams showing the operation section 50. Fig. 2A is an exterior appearance view. Fig. 2B is a cross-sectional view of a nozzle section. Fig. 2C shows the front end of the nozzle. Fig. 3 is a diagrammatic cross-sectional view showing the internal configuration of a pulsed flow applying part 60. The operation section 50 is a portion gripped with the practitioner's hand and operated by the practitioner for medical practice, as shown in Fig. 2A. The operation section 50 includes a nozzle section 52, a gripper section 58, and a pulsed flow applying part 60 as an ejection drive section, which are sequentially arranged from a medical practice target site when viewed with the operation section 50 facing the medical practice target site.

The nozzle section 52 includes an ejection tube 54 and a suction tube 14a, each of which is a hollow tube, as shown in Figs. 2B and 2C. In the present embodiment, the ejection tube 54 is, for example, a small-diameter tube through which the liquid L is ejected to a medical practice target site. The ejection tube 54 is inserted into the suction tube 14a, the inner diameter of which is greater than the outer diameter of the ejection tube 54. That is, the ejection tube 54 is disposed at the center of the nozzle section 52, and the suction tube 14a surrounds the ejection tube 54. An ejection port 54b of the ejection tube 54 and a suction port 14b of the suction tube 14a are roughly flush with each other in a basic setting but can be positionally adjusted.

The present embodiment has been described with reference to the case where the ejection tube 54 is inserted into the suction tube 14a, but this configuration is not necessarily employed. The ejection tube 54 and the suction tube 14a may be disposed side by side, or the suction tube 14a may be disposed at the center and the ejection tube 54 may surround the suction tube 14a. Still instead, the suction tube 14a may be an extension of the suction tube 14, which forms the tube group 15, or may communicate with the suction tube 14 via a separate tube.

The gripper section 58 includes a main body case 59, a suction adjuster 75, and a connection section 77. The main body case 59 is preferably formed of a molded part made, for example, of a plastic material and has a roughly cylindrical shape so deformed that a portion close to a medical practice target site slightly narrows. The gripper section 58 is a portion gripped mainly by the practitioner in many cases and is precisely operated for excision or fragmentation of living tissue at a medical practice target site with the ejection port 54b of the ejection tube 54 facing the medical practice target site. In this process, the practitioner passes the operation section 50 from the right hand to the left hand and vice versa and changes the way the operation section 50 is gripped for accurate medical practice in accordance with a variety of situations, such as provision of a secure field of view with the aid of a microscope or a magnifying glass, the direction in which a target site is excised, and other medical practice tools to be used.

The nozzle section 52 described above is arranged on one side of the main body case 59 of the gripper section 58, and the connection section 77 is provided on the other side of the main body case 59. The nozzle section 52 is so arranged as to be inclined to the axial line of the main body case 59 by a predetermined angle. The ejection direction of the liquid ejected through the nozzle section 52 is therefore inclined to the direction in which the practitioner grips the operation section 50 by the predetermined angle. As a result, the practitioner who performs medical practice can fully recognize a medical practice target site. In particular, when medical practice is performed by using a microscope or a magnifying glass, the front end of the main body case 59 or the nozzle section 52 does not block the field of view of the microscope or the magnifying glass. In particular, when the liquid is ejected downward, the inclined nozzle section 52 is effective.

The suction tube 14, which will be described later, is so connected to the connection section 77 that the suction tube 14 is, for example, fit in the connection section 77. In the present embodiment, the connection section 77 is further provided with a linkage section 78, and one end of the pulsed flow applying part 60 is removably attached to the linkage section 78 in fitting or rotating action or in any other action.

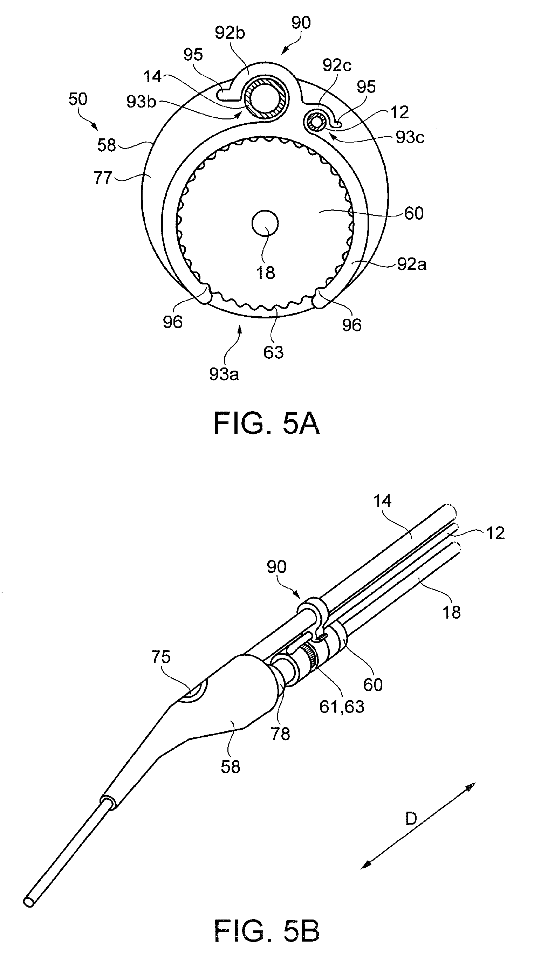

The pulsed flow applying part 60 has a roughly cylindrical shape and has, although not shown, a threaded end portion on one side that is linked with the linkage section 78. The supply tube 12 is connected to the pulsed flow applying part 60 in a position in the vicinity of the threaded end portion, and the cable 18 is connected to the end of the pulsed flow applying part 60 on the other side. The pulsed flow applying part 60 forms one cylindrical member. An outer circumferential grooved section 61 is formed along the outer surface of the roughly cylindrical shape of the pulsed flow applying part 60, and crest/trough-shaped locking reception sections 63 (see Fig. 5A), which is formed of crests having a predetermined height in the radial direction and formed at predetermined intervals in the circumferential direction, is provided at the bottom of the grooved section 61.

The pulsed flow applying part 60 includes a first case 70, a second case 71, a third case 72, a piezoelectric element 62, a diaphragm 64, an inlet channel 67, an outlet channel 68, and a pump chamber 66, as shown in Fig. 3. The second case 71 is a tubular member and has one end that forms a flange, and the flange faces the first case 70 and is bonded thereto. The other end of the second case 71 is bonded to the third case 72, and a cylindrical space is formed in the second case 71. The piezoelectric element 62 is disposed in the interior space of the second case 71.

The piezoelectric element 62 is a laminated piezoelectric element and forms an actuator. One end of the piezoelectric element 62 on the side facing the first case 70 is in contact with the diaphragm 64, and the other end of the piezoelectric element 62 is fixed to the third case 72. The cable 18 is connected to the other end of the piezoelectric element 62, and the piezoelectric element 62 receives, through the other end thereof, a drive signal transmitted from the controller 25 in the device main body section 20. The diaphragm 64 is formed of a disk-shaped metal thin film, and the periphery of the diaphragm 64 is fixed to the second case 71. The pump chamber 66, which is a space having a predetermined volume, is formed between the diaphragm 64 and the first case 70, and the volume changes in accordance with the action of the piezoelectric element 62 driven with the drive signal.

The inlet channel 67 and the outlet channel 68 are formed in the first case 70. The inlet channel 67 communicates with the side surface of the pump chamber 66. The supply tube 12 connected to the pulsed flow applying part 60 is connected to the inlet channel 67. The liquid L supplied from the device main body section 20 is therefore supplied via the supply tube 12 and the inlet channel 67 into the pump chamber 66. The outlet channel 68 communicates with a surface of the pump chamber 66, that is, the surface thereof perpendicular to the direction in which the piezoelectric element 62 is displaced. The ejection tube 54 in the nozzle section 52 is connected to the outlet channel 68.

The suction adjuster 75 is provided in the main body case 59 and in a portion where the outer diameter of the exterior of the main body case 59 slightly decreases, as shown in Figs. 2A and 3. In the present embodiment, the suction adjuster 75 is formed as an open hole section 75a, and one end of the hole section 75a communicates with the outside air and the other end thereof connects to the inner wall of the suction tube 14a. The interior of the suction tube 14a is sucked at predetermined pressure by the liquid suction mechanism 40 in the device main body section 20. The sucking action can therefore be initiated or terminated and suction force can be adjusted by opening or closing the hole section 75a of the suction tube 14a or changing the open area of the hole section 75a. That is, the practitioner who grips the main body case 59 adjusts the suction adjuster 75 by opening or closing the hole section 75a or adjusting the open area of the hole section 75a with the thumb or the index finger.

The present embodiment has been described with reference to the case where the suction adjuster 75 is formed as the open hole section 75a and the practitioner operates the suction adjuster 75 with a finger, but the suction adjuster 75 is not necessarily configured this way. The suction adjuster 75 may be operated, for example, with a slide switch or a foot switch.

The tube group 15, which includes the supply tube 12, the suction tube 14, and the cable 18, will now be described in detail with reference to Figs. 2A to 2C. The suction tube 14 is directly connected to the connection section 77, as shown in Fig. 2A. In the present embodiment, the suction tube 14 is preferably formed of a tube made, for example, of polyurethane, polyvinyl chloride, or any other synthetic resin. A tube made of polyurethane, polyvinyl chloride, or any other synthetic resin excels in tensile resistance and flexibility and has other advantages. The supply tube 12 is connected to the pulsed flow applying part 60 in the vicinity of one end thereof and preferably formed of a tube made, for example, of high-density polyethylene. A tube made of high-density polyethylene excels in water resistance and chemical resistance and has other advantages.

The cable 18 is connected to the other end of the pulsed flow applying part 60 and accommodates a power line and a signal line therein, and the exterior of the cable 18 is coated with a synthetic resin, such as silicon and polyvinyl chloride. Polyvinyl chloride excels in heat resistance, flame retardation, and environmental friendliness and has other advantages. Silicon has properties as an elastic material maintained over a wide temperature range and shows excellent resistance to ozone, humidity, electrical insulation, hot water, and chemicals.

In the present embodiment, the outer diameter of each of the tubes in the tube group 15 is so set that the supply tube 12 has the smallest outer diameter ranging from about 2 to 3 mm and the cable 18 and the suction tube 14 have greater outer diameters or the diameters of the supply tube 12, the cable 18, and the suction tube 14 increase in this order. During medical practice, the supply tube 12 is roughly always filled with the liquid L, and the liquid M containing part of fragmentated tissue flows inside the suction tube 14 in an intermittent or continuous manner. The cable 18 accommodates a power line and a signal line therein. The tubes in the tube group 15 therefore differ from one another in terms of rigidity.

The rigidity used herein refers to the magnitude of force required to bend or twist each of the tubes or the magnitude of reaction force produced by each of the tubes when the tube attempts to restore the initial shape. Further, the degree of rigidity is determined by the outer diameter and the thickness of each of the tubes, whether or not the liquid L or M flows through the tube, the content accommodated in the tube, and other factors. The tube group 15 including the supply tube 12, the suction tube 14, and the cable 18 is connected between the device main body section 20 and the operation section 50, and each of the tubes has a length of several meters. Each of the supply tube 12, the suction tube 14, and the cable 18 forms one cylindrical member.

### Action of liquid ejection device

The action of the liquid ejection device 10 will next be described with reference to Figs. 1 to 3. In the liquid ejection device 10 having the configuration described above, when the power switch 24 on the device main body section 20 shown Fig. 1 is flipped on, electric power is supplied to the controller 25. The practitioner grips the operation section 50, orients the nozzle section 52 toward a medical practice target site, and flips on the ejection switch 26.

When the ejection switch 26 is flipped on, the supply pump 34 in the liquid supply mechanism 30 is activated, extracts the liquid L stored in the liquid storage 32 through the liquid acquisition tube 34a, and causes the liquid L to flow to the electromagnetic valve 38. When the controller 25 opens the electromagnetic valve 38, the liquid L travels as a fluid inside the supply tube 12 arranged in the device main body section 20. In this process, the supply flowmeter 37 detects the flow rate of the liquid L traveling inside the supply tube 12 and outputs the detected flow rate to the controller 25. The action of the supply flowmeter 37 allows adjustment of the quantity and pressure of the liquid L delivered from the device main body section 20. Further, the quantity and pressure of the liquid L are displayed in the display section 22 on the device main body section 20.

The liquid L delivered from the device main body section 20 travels inside the supply tube 12, which forms the tube group 15, which is connected between the device main body section 20 and the pulsed flow applying part 60, and reaches the pulsed flow applying part 60. The liquid L having reached the pulsed flow applying part 60 travels via the inlet channel 67 and fills the pump chamber 66. The volume of the pump chamber 66 changes in accordance with the action of the piezoelectric element 62. That is, the piezoelectric element 62 expands or contracts in the direction indicated by the arrow A or B in Fig. 3 in response to the drive signal transmitted from the controller 25 in the device main body section 20 via the cable 18. When the piezoelectric element 62 expands in the direction indicated by the arrow A, the diaphragm 64 is pressed and bent by the piezoelectric element 62 in the direction indicated by the arrow A in Fig. 3, resulting in a decrease in the volume of the pump chamber 66. The liquid L in the pump chamber 66 is therefore pushed out of the pump chamber 66 and travels to the outlet channel 68. The liquid L traveling through the outlet channel 68 travels inside the ejection tube 54, which communicates with the outlet channel 68, in the nozzle section 52 and is ejected through the ejection port 54b.

When the piezoelectric element 62 contracts in the direction indicated by the arrow B, the diaphragm 64 is bent in the B direction in Fig. 3 in synchronization with the action of the piezoelectric element 62, resulting in an increase in the volume of the pump chamber 66. The liquid L is therefore supplied into the pump chamber 66 through the inlet channel 67. That is, driving the piezoelectric element 62 at a predetermined frequency allows the liquid L supplied from the liquid supply mechanism 30 in the device main body section 20 to be ejected through the ejection tube 54 in the form of pulsed jet flow. The liquid L ejected through the ejection tube 54 in the form of pulsed jet flow excises or fragmentates tissue at a medical practice target site.

At this point, the liquid M containing part of the fragmentated tissue and excess liquid L that blocks the field of vision during the medical practice are also present at the medical practice target site. The following description will be made on the assumption that the liquid L once ejected through the ejection tube 54 is entirely changed to the liquid M for ease of description. The liquid M is sucked by the liquid suction mechanism 40.

When the practitioner flips the ejection switch 26 on, not only is the supply pump 34 activated, but also the suction pump 44 in the liquid suction mechanism 40 is activated roughly at the same time. The suction pump 44 performs sucking action in the direction indicated by the arrow B in Fig. 3 in the channel including the suction tubes 14 and 14a. As a result, the pressure in the suction tube 14a in the operation section 50 goes negative. At this point, when the hole 75a is open, the outside air is sucked through the hole 75a, whereas when the practitioner closes the hole 75a, the liquid M present around the suction port 14b of the suction tube 14a is sucked.

The operation section 50 has the suction adjuster 75, as described above. The suction adjuster 75 is formed as the open hole section 75a. The practitioner opens or closes the hole section 75a or changes the open area with the thumb or the index finger while gripping the main body case 59. The practitioner can thus perform and terminate the suction of the liquid M and adjust the quantity of sucked liquid M. The liquid M can be sucked in a state in which the liquid L is ejected through the ejection port 54b or in a state in which the ejection switch 26 is flipped off to stop ejecting the liquid L through the ejection port 54b. The adjustment of the quantity of sucked liquid M is preferably made in accordance with how much the liquid M stays at the medical practice target site or how well the field of vision is ensured during the medial practice.

The liquid M sucked through the suction port 14b travels through the suction tube 14a in the operation section 50 and the suction tube 14 connected to the suction tube 14a into the suction tube 14 in the device main body section 20. The liquid M having traveled into the device main body section 20 travels through the suction flowmeter 46, the suction pump 44, and the drainage tube 44a and is stored in the drainage tank 42. The suction flowmeter 46 detects the flow rate of the liquid M traveling inside the suction tube 14 and outputs the detected flow rate to the controller 25. The suction flowmeter 46 senses whether the suction is being reliably performed, how much of liquid M is stored in the drainage tank 42, and other conditions.

### Support tool

### First Embodiment

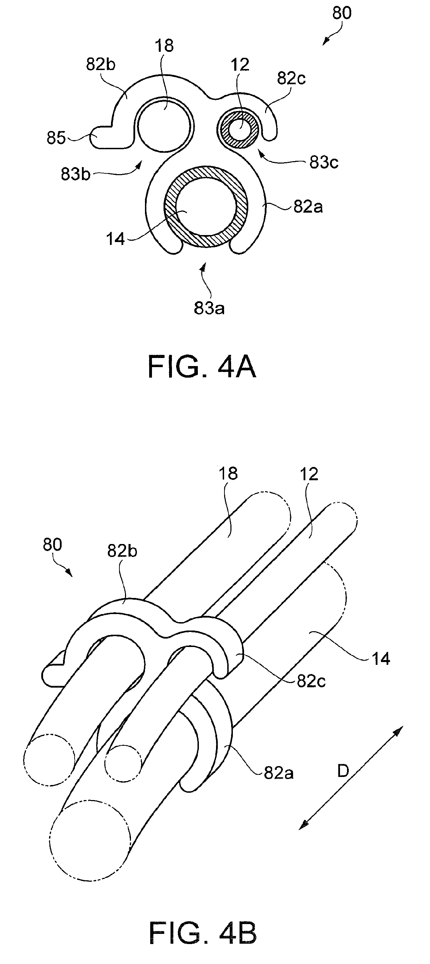

A support tool according to a first embodiment will be described with reference to Figs. 4A and 4B. Figs. 4A and 4B describe the support tool according to the first embodiment. Fig. 4A is a cross-sectional view showing the support tool according to the first embodiment and the tube group, and Fig. 4B is a perspective view showing the relationship between the support tool according to the first embodiment and the tube group.

A support tool 80 according to the first embodiment has a function of supporting the tube group 15 described above as the cylindrical members. The following description will be made with reference to a case where the tube group 15 is formed of three tubes, the supply tube 12, the suction tube 14, and the cable 18.

The support tool 80 is preferably made, for example, of a synthetic resin, such as a plastic material, and has support sections 82a, 82b, and 82c, which are integrated with one another in a molding process, as shown in Fig. 4A. The support sections 82a, 82b, and 82c have arcuate shapes (ring shapes having open portions) having openings 83a, 83b, and 83c, each of which has a predetermined dimension and is open outward. In the first embodiment, the support section 82a corresponds to the suction tube 14, which has the greatest outer diameter, the support section 82b corresponds to the cable 18, which has the second greatest outer diameter, and the support section 82c corresponds to the supply tube 12, which has the smallest outer diameter.

The support section 82a supports the suction tube 14, which is inserted through the opening 83a, in such a way that the suction tube 14 is immobile in the axial direction thereof (direction indicated by arrow D in Fig. 4B). In this case, for example, the inner diameter of the support section 82a is set to be smaller than the outer diameter of the suction tube 14 to achieve what is called a shrink-fit state. The immobile state used herein includes a state in which the suction tube 14 is completely fixed and a state in which the suction tube 14 slightly moves when axial force acts thereon. That is, the suction tube 14 only needs not to readily move.

The support section 82b supports the cable 18, which is inserted through the opening 83b, in such a way that the cable 18 is movable in the axial direction thereof. In this case, for example, the inner diameter of the support section 82b is set to be greater than the outer diameter of the cable 18 to achieve a state in which a gap is present between the support section 82b and the cable 18. Similarly, the support section 82c supports the supply tube 12, which is inserted through the opening 83c, in such a way that the supply tube 12 is movable in the axial direction thereof. In this case, for example, the inner diameter of the support section 82c is set to be greater than the outer diameter of the supply tube 12 to achieve a state in which a gap is present between the support section 82c and the supply tube 12. The support states described above are adjusted in accordance with the outer diameters of the tubes in the tube group 15, the inner diameters of the support sections 82a, 82b, and 82c, which support the tube group 15, the ratios of the dimensions of the openings 83a, 83b, and 83c to the outer diameters of the respective tubes in the tube group 15, and other factors.

In the first embodiment, a guiding section 85 is formed at an end of the support section 82b or the end thereof forming the opening 83b. The guiding section 85 has a receiving shape that so extends as to form an inclined surface that is open in the direction in which the cable 18 is inserted. The guiding section 85 therefore assists the cable 18, when it is inserted, in being guided toward the opening 83b.

The support tool 80 bundles the supply tube 12, the suction tube 14, and the cable 18, which form the tube group 15, as shown in Fig. 4B. Fig. 4B shows a state in which the tubes are bundled at a single point, but the support tool 80 is actually formed of a plurality of support tools 80 along the length direction of the tube group 15 at predetermined intervals. In this configuration, the suction tube 14 is immobile relative to the support tools 80, and the supply tube 12 and the cable 18 are movable relative to the support tools 80 in the axial direction, that is, the length direction of the tubes (direction indicated by arrow D in Fig. 4B).

Advantageous effects provided by the first embodiment will be described below.
(1) The support tools 80 described above are arranged at predetermined intervals in the length direction of the tube group 15. The support tools 80 can bundle the supply tube 12, the suction tube 14, and the cable 18, which form the tube group 15. The support tools 80 can therefore prevent the supply tube 12, the suction tube 14, and the cable 18 from coming apart in the radial direction. As a result, the liquid ejection device 10 provided by the first embodiment includes the operation section 50 having the support tools 80 that allow the practitioner to perform accurate operation with excellent operability.
(2) Each of the support tools 80 described above uses the support section 82a to support the suction tube 14, which has the greatest outer diameter and highest rigidity, as a first cylindrical member in such a way that the suction tube 14 is immobile in the length direction thereof. The support tool 80 further uses the support sections 82b and 82c to support the cable 18 and the supply tube 12, each of which has a smaller outer diameter and lower rigidity than the suction tube 14, as second cylindrical members in such a way that the cable 18 an the supply tube 12d are movable in the length direction thereof. Therefore, even the tube group 15, which is formed of a plurality of tubes different from one another in terms of rigidity and behavior, basically curves and behaves in accordance with the curvature and behavior of the suction tube 14. As a result, differences in the curvature and behavior in the tube group 15 that occur when the practitioner operates the operation section can be reduced, whereby variation in reaction force produced by the tubes in the tube group 15 can be reduced. Therefore, the liquid ejection device 10 provided by the first embodiment includes the operation section 50 having the support tools 80 that allow the practitioner to perform accurate operation with excellent operability.

### Second Embodiment

A support tool according to a second embodiment will be described with reference to Figs. 5A and 5B. Figs. 5A and 5B describe the support tool according to the second embodiment. Fig. 5A is a plan view of the support tool according to the second embodiment, and Fig. 5B is a perspective view showing the relationship between the support tool according to the second embodiment and the operation section.

A support tool 90 according to the second embodiment has a function of supporting part of the pulsed flow applying part 60 and the tube group 15, which are connected as the cylindrical members to the operation section 50. The following description will be made with reference to a case where the tube group 15 is formed of three tubes, the supply tube 12, the suction tube 14, and the cable 18.

The support tool 90 is preferably made, for example, of a synthetic resin, such as a plastic material, and has support sections 92a, 92b, and 92c, which are integrated with one another in a molding process, as shown in Fig. 5A. The support sections 92a, 92b, and 92c have arcuate shapes (ring shapes having open portions) having openings 93a, 93b, and 93c, each of which has a predetermined dimension and is open outward.

The support section 92a corresponds to the pulsed flow applying part 60, and locking sections 96, each of which protrudes toward the center of the arc of the support section 92a, are formed at opposite ends of the opening 93a of the support section 92a. To attach the support section 92a to the pulsed flow applying part 60, the support section 92a of the support tool 90 is fit in the grooved section 61 along the outer surface of the roughly cylindrical shape of the pulsed flow applying part 60 shown in Fig. 2A. Axial movement of the pulsed flow applying part 60 is therefore restricted. Further, the locking sections 96 of the support section 92a, which protrude toward the center of the arc of the support section 92a, lock with the crest/trough-shaped locking reception sections 63, which are formed at the bottom of the grooved section 61, in a stepwise manner. The support tool 90 is therefore positioned in the circumferential direction in a stepwise manner.

The support section 92b corresponds to the suction tube 14, and the support section 92c corresponds to the supply tube 12. The support section 92b supports the suction tube 14, which is inserted through the opening 93b, in a state in which the suction tube 14 is movable in the axial direction thereof. The support section 92c supports the supply tube 12, which is inserted through the opening 93c, in a state in which the supply tube 12 is movable in the axial direction thereof. Guiding sections 95 are formed at ends of the openings 93b and 93c of the support sections 92b and 92c. The guiding sections 95 have receiving shapes that so extend as to form inclined surfaces that are open in the directions in which the suction tube 14 and the supply tube 12 are inserted. The guiding sections 95 therefore assist the suction tube 14 and the supply tube 12, when they are inserted, in being guided toward the openings 93b and 93c.

The support tool 90 supports and bundles the pulsed flow applying part 60 as a first cylindrical member, the supply tube 12, and the suction tube 14, which are connected to the operation section 50, as shown in Fig. 5B. In this configuration, the suction tube 14 and the supply tube 12 are movable relative to the support tool 90 in the axial direction, that is, the length direction of the tubes (direction indicated by arrow D in Fig. 5B).

Advantageous effects provided by the second embodiment will be described below.

According to the configuration described above, the support tool 90 is fixed to the position of a component of the operation section 50 or the device main body section 20, that is, a predetermined position. The support tool 90 can support the tube group 15 in that position in such a way that the tube group 15 is movable in the axial direction thereof but immobile in the radiation direction thereof. Therefore, variation in the positions of the tubes in the tube group 15 in the radial direction can be suppressed and a situation in which the tubes come apart in the radial direction can be avoided. As a result, differences in the behavior of the tubes in the tube group 15 can be reduced, whereby variation in reaction force produced by the plurality of tubes in the tube group 15 can be reduced. The liquid ejection device 10 provided by the second embodiment therefore includes the operation section 50 having the support tool 90 that allows the practitioner to perform accurate operation with excellent operability.

According to the configuration of the support tool 90 described above, one of the arcuate support sections of the support tool 90 is fit in the grooved section provided around the circumference of the pulsed flow applying part 60 to achieve a state in which the pulsed flow applying part 60 is movable along the grooved section 61 in the circumferential direction but immobile in the axial direction. Further, when the locking sections 96, which protrude inward from the support section 92a, engage with the locking reception sections 63, which are formed at predetermined intervals in the grooved section 61 of the pulsed flow applying part 60, the support tool 90 can be held in a predetermined position in the circumferential direction. The support tool 90 can therefore support the tube group 15 with a radial load reduced even in a state in which the tube group 15 is arranged in an arbitrary position relative to the pulsed flow applying part 60.

The embodiments of the invention have been described above, and a variety of changes can be made to the embodiments described above to the extent that the changes do not depart from the substance of the invention. For example, variations different from the embodiments described above are as follows.

The above embodiments have been described with reference to the case where the tube group 15 is formed of three tubes, the supply tube 12, the suction tube 14, and the cable 18, but the tube group 15 is not necessarily formed of three tubes. The liquid ejection device 10 does not necessarily have the suction function, and the tube group 15 may therefore be formed of two tubes, the supply tube 12 and the cable 18. Still instead, the liquid ejection device 10 may eject a plurality of liquids L, and two or more supply tubes 12 may therefore be provided. Further, to enhance the suction performance of the liquid ejection device 10, two or more suction tubes 14 may be provided. A liquid ejection device 10 including an operation section 50 capable of accurate operation can be provided by arrangement of a plurality of tubes having different degrees of rigidity in accordance with the spirit of the invention described above.

In each of the embodiments described above, the configuration in which the piezoelectric element 62 is used as the pulsed flow applying part 60 is disclosed, but the piezoelectric element 62 is not necessarily used. An ejection mechanism based on a thermal jet method using a laser, a heater, and other components may be used. Further, the pulsed flow applying part 60 may be arranged outside the operation section 50, but the ejection tube 54 may be arranged in the operation section 50. Still further, continuous flow may be ejected as well as pulsed flow.

Since it is conceivable that the tube group 15 is exchanged in every surgery in consideration of contamination and damage in the surgery or any other situation, the tubes in the tube group 15 may be configured to be attachable and detachable individually or in the form of a kit to and from the device main body section 20 and the operation section 50. Further, the material and the quality of the tube group described in the above embodiments are presented by way of example and are not necessarily employed.

The above embodiments have been described with reference to the case where the states in which the support sections 82a, 82b, and 82c of the support tool 80 support the tube in the tube group 15 are adjusted by the inner diameters of the support sections 82a, 82b, and 82c and the dimensions of the openings 83a, 83b, and 83c, but the adjustment is not necessarily made as described above. The adjustment can be made, for example, on the basis of the modulus of elasticity of a plastic material or any other synthetic resin material of which the support tool 80 is made, differences in rigidity and elasticity according to the shapes of the support sections, the frictional coefficients of the portions where the support sections 82a, 82b, and 82c are in contact with outer circumferential portions of the tubes in the tube group 15, employment of a skillfully configured shape and structure for slip prevention or any other purpose, and insertion of separate members.

In the embodiments described above, the state in which each of the support sections 82a, 82b, and 82c of the support tool 80 supports the corresponding tube in the tube group 15 is the state in which the tube and other components are movable or immobile relative to the support tool 80. Instead, the support tool 80 may be movable or immobile relative to the tube group 15 and other components. In this case, even when the tube group 15 and other components are curved, the support tool 80 is immobile relative to part of the tubes, that is, fixed thereto, whereby the possibility of a change in the position of the support tool 80, that is, the possibility of movement of the support tool 80 can be lowered.

## Claims

1. A support tool adapted to be used with a liquid ejection device including an operation section operated by an operator and a device main body section and supports a plurality of cylindrical members including a tube group connected to the operation section and the device main body section, the support tool comprising:
a first support section capable of supporting a first cylindrical member among the plurality of cylindrical members in such a way that the first cylindrical member is immobile in an axial direction thereof; and
second support sections capable of supporting second cylindrical members different from the first cylindrical member in such a way that the second cylindrical members are movable in axial directions thereof,
wherein the first support section and the second support sections are integrated with one another.

2. The support tool according to claim 1,
wherein the first cylindrical member is at least one of a tube or a cable having a greatest outer diameter and a tube or a cable having highest rigidity in the tube group.

3. The support tool according to claim 1 or 2,
wherein the first support section has a first opening through which the first cylindrical member is inserted,
each of the second support sections has a second opening through which the corresponding second cylindrical member is inserted,
an inner diameter of the first support section is smaller than an outer diameter of the first cylindrical member, and
an inner diameter of each of the second support sections is greater than an outer diameter of the corresponding second cylindrical member.

4. The support tool according to claim 3,
wherein a second opening is formed of a first portion and a second portion of the corresponding second support section,
a protrusion extending in a direction away from the second portion is formed in the first portion, and
a surface of the protrusion forms an inclined surface extending toward the second opening in a direction in which the corresponding second cylindrical member is inserted.

5. The support tool according to any one of the preceding claims,
wherein the first cylindrical member is part of a pulsed flow applying unit connected to the operation section, and
the second cylindrical members are formed of the tube group.

6. The support tool according to claim 5,
wherein the first cylindrical member has a cylindrical portion having an outer circumferential portion around which a grooved section is formed, and
the first support section has a locking section fittable to the grooved section.

7. An operation section adapted to be used with a liquid ejection device that ejects liquid and operated by an operator, the operation section comprising:
a gripper section;
a tubular nozzle section that is provided in the gripper section and ejects the liquid;
a tube group which is connected to the gripper section and through which the liquid flows; and
the support tool according to any one of the preceding claims, which supports at least one tube in the tube group.

8. A liquid ejection device comprising:
the operation section according to claim 7;
an ejection drive section that ejects liquid through a nozzle section provided in the operation section;
a liquid supply mechanism that supplies the ejection drive section with the liquid;
a liquid suction mechanism capable of sucking the liquid ejected through the nozzle section;
a device main body section that outputs a drive signal to the ejection drive section; and
a cable connected between the ejection drive section and the device main body section.
